# EUROPEAN PATENT APPLICATION

(11) **EP 0 925 788 A1**
(43) Date of publication of application: **30.06.1999**
(21) Application number: 98907187.3
(22) Date of filing: 12.03.1998
(51) Int. Cl.: A61K 31/557

(54) **DRUGS FOR RELIEVING DIABETIC VASCULAR LESION**

(30) Priority: 13.03.1997 JP 5914697
(71) Applicant: TORAY INDUSTRIES, INC., Tokyo 103-8666 (JP)
(72) Inventor: KURUMATANI, Hajimu, Kamakura-shi Kanagawa 248-0034 (JP); ANNOH, Shigeyasu, Kamakura-shi Kanagawa 248-0036 (JP); KOIKE, Hiroshi, Mishima-shi Shizuoka 411-0033 (JP)
(74) Representative: Linn, Samuel Jonathan
(86) International application number: JP9801029
(87) International publication number: WO9840073

(57) **Abstract**

The present invention provides a diabetic angiopathy improving agent comprising a prostaglandin I derivative such as beraprost as an active component, and a method of preventing diabetic angiopathy by using the improving agent. This medicine exhibits excellent effects as a diabetic angiopathy inhibiting agent in both oral administration and parenteral administration.

## Description

### Technical Field

The present invention relates to diabetic angiopathy improving agent comprising a prostaglandin I derivative or a salt thereof as an active ingredient.

### Background Art

Known vascular lesions of diabetic include diabetic macroangiopathy and diabetic microangiopathy. The diabetic macroangiopathy mainly means arterioscleotic lesions which occur in the aorta, and the diabetic microangiopathy means arteriola or capillary lesions peculiar to diabetic, which occur in the small vessels of the retina and the kidney and so on. Diabetic angiopathy is known to cause decreases in the vasoconstriction by a vasoconstrictor, vasodilation, anti-thrombogenic activity of the vascular endothelial cells, etc. Pathologically, the diabetic microangiopathy causes thickening of the arterial walls and the capillary basement membranes, and deposition of a PAS positive substance, and is considered as being related to occurrence or worsening of diabetic retinopathy, diabetic nephropathy and diabetic neuropathy. On the other hand, the diabetic macroangiopathy represents vascular lesions which occur in the aorta and relatively large arterial branches therefrom, and is known to take a form similar to usual atherosclerosis but occur earlier and progress rapidly.

It is also considered that longstanding hyperglycemia readily causes diabetic angiopathy, and that onset and progress of angiopathy can be delayed by controlling blood sugar to be close to a normal state. Therefore, the blood sugar value is generally controlled by alimentary therapy and administration of insulin or an oral hypoglycemic. However, such therapy exhibits insufficient effects, and more effective therapy is demanded.

Prostanoic acid derivatives are known to have various biological activities, but are classified to some groups according to modification of a five-member ring comprising C-8 to C-12. Of such derivatives, a compound having epoxide between the 6- and 9-positions is referred to as "PGI", and prostaglandin I₂ (PGI₂, prostacyclin) is known as a representative. PGI₂ is known as a substance having the action of suppressing platelet aggregation and the action of dilating peripheral vessels (refer to Nature, Vol. 268, p. 688, 1976). However, since PGI₂ has an unstable exoenol structure, it is very unstable even in a neutral aqueous solution and is converted into 6-oxo PGF1α having substantially no biological activity, without maintaining the biological action thereof. As compounds in which such instability is significantly improved, Japanese Examined Patent Publication Nos. 2-12226, 2-57548 and 1-53672 disclose PGI₂ derivatives having a skeleton in which the structure of the exoenol ether moiety characteristic of the structure of PGI₂ is converted into an inter-m-phenylene structure. Other known compounds in which the stability of prostaglandin I is improved include ataprost, iloprost, clinprost, ciprostene, naxaprostene, taprostene, cicaprost, pimilprost, CH-169, and CS570 (refer to Gendai-Iryosha, "Generals of Prostaglandin" No. 1, p. 123, 1994; New Drugs of Tomorrow, p. 15-IV-185, 1996; New Drugs of Tomorrow, p. 15-III-551, 1996). However, it is unknown that these prostaglandin I derivatives improve diabetic angiopathy.

An object of the present invention is to provide an agent for preventing or curing diabetic angiopathy.

### Disclosure of Invention

The present invention provides a diabetic angiopathy improving agent comprising, as an active ingredient, a prostaglandin I derivative, preferably a 4,8-inter-m-phenyleneprostaglandin I derivative represented by the following formula (I), or a pharmacologically acceptable salt thereof, and a method of preventing or curing diabetic angiopathy. 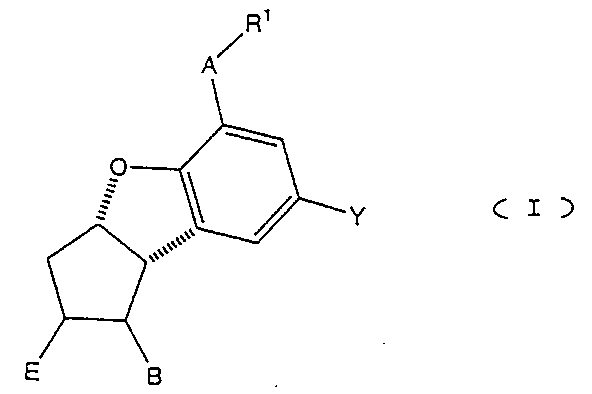 wherein R¹ represents the following:
(A) COOR² wherein R² is:
   1) hydrogen or a pharmacologically acceptable cation;
   2) straight chain alkyl having 1 to 12 carbon atoms, or branched alkyl having 3 to 14 carbon atoms;
   3) -Z-R³
      wherein Z is a valence bond or straight chain or branched alkylene represented by CₜH₂ₜ wherein t represents an integer of 1 to 6, and R³ represents cycloalkyl having 3 to 12 carbon atoms or substituted cycloalkyl having 3 to 12 carbon atoms and 1 to 3 substituents R⁴ which is hydrogen or alkyl having 1 to 5 carbon atoms;
   4) -(CH₂CH₂O)ₙCH₃
      wherein n is an integer of 1 to 5;
   5) -Z-Ar¹
      wherein Z is defined as the same as the above, and Ar¹ is phenyl, α-naphthyl, β-naphthyl, 2-pyridyl, 3-pyridyl, 4-pyridyl, α-furyl, β-furyl, α-thienyl, β-thienyl or substituted phenyl (wherein a substituent is at least one of chlorine, bromine, fluorine, iodine, trifluoromethyl, alkyl having 1 to 4 carbon atoms, nitro, cyano, methoxy, phenyl, phenoxy, p-acetamidobenzamide, -CH=N-NH-C(=O)-NH₂, -NH-C(=O)-Ph, -NH-C(=O)-CH₃ and -NH-C(=O)-NH₂);
   6) -CₜH₂ₜCOOR⁴
      wherein CₜH₂ₜ and R⁴ are defined as the same as the above;
   7) -CₜH₂ₜN(R⁴)₂
      wherein CₜH₂ₜ and R⁴ are defined as the same as the above;
   8) -CH(R⁵)-C(=O)-R⁶
      wherein R⁵ is hydrogen or benzoyl, and R⁶ is phenyl, p-bromophenyl, p-chlorophenyl, p-biphenyl, p-nitrophenyl, p-benzamidophenyl, or 2-naphthyl;
   9) -CₚH₂ₚ-W-R⁷
      wherein W is -CH=CH-, -CH=CR⁷ or -C≡C-, and R⁷ is hydrogen or straight chain or branched alkyl or aralkyl having 1 to 30 carbon atoms, and p is an integer of 1 to 5; or
   10) -CH(CH₂OR⁸)₂
      wherein R⁸ is alkyl or acyl having 1 to 30 carbon atoms;
(B) -CH₂OH;
(C) -C(=O)N(R⁹)₂
   wherein R⁹ is hydrogen, straight chain alkyl having 1 to 12 carbon atoms, branched alkyl having 3 to 12 carbon atoms, cycloalkyl having 3 to 12 carbon atoms, cycloalkylalkylene having 4 to 13 carbon atoms, phenyl, substituted phenyl (wherein the substituent is defined as the same as in (A) 5)), aralkyl having 7 to 12 carbon atoms, or -SO₂R¹⁰ wherein R¹⁰ is alkyl having 1 to 10 carbon atoms, cycloalkyl having 3 to 12 carbon atoms, phenyl, substituted phenyl (the substituent is defined as the same as in (A) 5)), or aralkyl having 7 to 12 carbon atoms, two R⁹ groups may be the same or different, and when one of the R⁹ groups is -SO₂R¹⁰, the other R⁹ is not -SO₂R¹⁰; or
(D) -CH₂OTHP (THP is a tetrahydropyranyl group);
A is the following:
1) -(CH₂)ₘ-;
2) -CH=CH-CH₂-;
3) -CH₂-CH=CH-;
4) -CH₂-O-CH₂-;
5) -CH=CH-;
6) -O-CH₂-; or
7) -C≡C-;
   wherein m represents an integer of 1 to 3;
   Y is hydrogen, alkyl having 1 to 4 carbon atoms, chlorine, bromine, fluorine, formyl, methoxy or nitro;
   B is -X-C(R¹¹)(R¹²)OR¹³
   wherein R^{1¹} is hydrogen, alkyl having 1 to 4 carbon
atoms; R¹³ is hydrogen, acyl having 1 to 14 carbon atoms, aroyl having 6 to 15 carbon atoms, tetrahydropyranyl, tetrahydrofuranyl, 1-ethoxyethyl, or t-butyl; X is the following:
1) -CH₂-CH₂-;
2) -CH=CH-; or
3) -C≡C-; and
   R¹² is the following:
   1) straight chain alkyl having 1 to 12 carbon atoms, or branched alkyl having 3 to 14 carbon atoms;
   2) -Z-Ar²
      wherein Z is the defined as the same as the above, and Ar² represents phenyl, α-naphthyl, β-naphthyl, or phenyl substituted by at least one chlorine, bromine, fluorine, iodine, trifluoromethyl, alkyl having 1 to 4 carbon atoms, nitro, cyano, methoxy, phenyl or phenoxy;
   3) -CₜH₂ₜOR¹⁴
      wherein CₜH₂ₜ is defined as the same as the above, and R¹⁴ represents straight chain alkyl having 1 to 6 carbon atoms, branched alkyl having 3 to 6 carbon atoms, phenyl, phenyl substituted by at last one chlorine, bromine, fluorine, iodine, trifluoromethyl, alkyl having 1 to 4 carbon atoms, nitro, cyano, methoxy, phenyl or phenoxy, cyclopentyl, cyclohexyl, or cyclopentyl or cyclohexyl substituted by 1 to 4 straight chain alkyl groups having 1 to 4 carbon atoms;
   4) -Z-R³
      wherein Z and R³ are defined as the same as the above;
   5) -CₜH₂ₜ-CH=C(R¹⁵)R¹⁶
      wherein CₜH₂ₜ is defined as the same as the above, and R¹⁵ and R¹⁶ each represent hydrogen, methyl, ethyl, propyl, or butyl; or
   6) -CᵤH₂ᵤ-C≡C-CR¹⁷
      wherein u is an integer of 1 to 7, CᵤH₂ᵤ represents straight chain or branched alkylene, and R¹⁷ represents straight chain alkyl having 1 to 6 carbon atoms;
      E is hydrogen or -OR¹⁸
      wherein R¹⁸ represents acyl having 1 to 12 carbon atoms, aroyl having 7 to 15 carbon atoms, or R² (wherein R² is defined as the same as the above); and
      the formula represents the d, l or dl form.

### Best Mode for Carrying Out the Invention

Prostaglandin I derivatives include prostaglandin I₁, I₂ and I₃ derivatives, and preferably, 4,8-inter-m-phenylene prostaglandin I derivatives or pharmacologically acceptable salts thereof can be used as active components of a diabetic angiopathy improving agent.

As prostaglandin I derivatives, beraprost or salts thereof are particularly preferable. Iloprost, clinprost, ataprost, ciprostene, naxaprostene, taprostene, cicaprost, CH-169, SM-10902 can also be used for the improving agent of the present invention.

The compounds represented by the formula (I) can be produced, for example, by the method disclosed in Japanese Examined Patent Publication No. 1-53672.

Oral or parenteral administration of a prostaglandin I derivative of the present invention improves diabetic macroangiopathy and diabetic microangiopathy. The prostaglandin I derivatives of the present invention also improve arterial sclerosis such as atherosclerosis and the like, and ischemic heart diseases and cerebrovascular diseases caused by arterial sclerosis. The derivatives also exhibit the effect of significantly improving diabetic retinopathy, diabetic nephropathy and diabetic neuropathy.

The compound represented by the formula (I) of the present invention is administered 1 to 3 times a day in a dose of 0.01 to 100 mg/adult.

Although the diabetic angiopathy improving agent of the present invention may contain at least one compound represented by the formula (I) or a salt thereof, the agent can also be orally administered in the form of a solid containing the additives below.

Examples of such additives include an excipient such as starch, lactose, sucrose, glucose, mannitol, potassium carbonate, calcium sulfate, or the like; a binder such as starch, dextrin, gum arabic, tragacanth, methyl cellulose, gelatin, polyvinyl pyrrolidone, polyvinyl alcohol, or the like; a disintegrator such as starch, polyvinyl pyrrolidone, crystalline cellulose, or the like; a lubricant such as magnesium stearate, talc, or the like; a colorant; a flavor; and the like.

The compounds represented by the formula (I) of the present invention can be used in various forms. Examples of the forms include conventional forms such as a tablet, a sugar-coated tablet, a powder, granules, a troche, a capsule, a pill, a syrup, and the like.

The compounds may also be parenterally administered in the form of a sterilized solution, and another solute such as sodium chloride, glucose, or the like can also be used in an amount sufficient for making the solution isotonic.

The diabetic angiopathy inhibiting agent of the present invention exhibits excellent medical effects either in oral administration or parenteral administration.

### [Examples]

Description will now be made of the diabetic angiopathy improving action of prostaglandin I compounds, particularly compounds represented by the formula (I) with reference to the beraprost sodium below (referred to as "BPS" hereinafter), but the compounds are not limited to this.

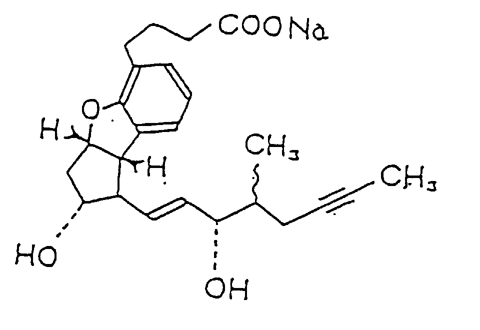

### Example 1

### Test of vasoconstriction in experimental diabetic model:

Diabetic contraction reaction disorder of small vessels was investigated by using a streptozotocin-induced diabetic rat model widely used for research of experimental diabetes lesions. Streptozotocin was intravenously administered to 8-week old SD rats to induce diabetes, and 2 weeks after administration of sterptozotocin, compound I was continuously orally administered to the rats twice a day for 2 weeks. Then, the mesenterium was extracted from each of the rats and used as a perfusion specimen for determining the vasoconstriction due to methoxamine as a vasoconstrictor as an increase (mmHg) in perfusion pressure. As a result, it was found that compound I significantly suppresses the significant decrease in contraction reaction of the small arteries, which was due to diabetes, and has the effect of curing diabetic angiopathy (Table 1).

**Table 1**

| | Methoxamine | |
|---|---|---|
| | 0.003 mM | 0.01 mM |
| Normal | 30.2 ± 7.9°° | 91.2 ± 16.4°° |
| Control | 3.5 ± 1.0 | 28.8 ± 2.9 |
| BPS 0.03 mg/kg | 4.5 ± 1.0 | 30.8 ± 3.5 |
| BPS 0.1 mg/kg | 11.3 ± 1.8°° | 43.5 ± 4.0° |
| Each value is average ± standard error. statistical significant difference: °; p < 0.05, °°; p < 0.01 vs. control. | | |

### Example 2

### Test of mesenteric relaxation reaction in experimental diabetic model

Vasorelaxation reaction disorder in diabetes was investigated by using a streptozotocin-induced diabetic rat model. Streptozotocin was intravenously administered to 8-week old SD rats to induce diabetes, and 2 weeks after administration of sterptozotocin, compound I was continuously orally administered to the rats twice a day for 2 weeks. Then, the mesenterium was extracted from each of the rats and used as a perfusion specimen. After the perfusion pressure was increased by using methoxamine as a vasoconstrictor, vasorelaxation reaction by acetylcholine was determined as a reduction of perfusion pressure. As a result, it was found that compound I significantly suppresses the significant decrease in relaxation reaction of the small arteries, which was due to diabetes, and has the effect of curing diabetic angiopathy (Table 2).

**Table 2**

| | Acetylcholine | | | |
|---|---|---|---|---|
| | 10 nM | 30 nM | 100 n | 1000 nM |
| Normal | 32.1 ± 7.1°° | 50.2 ± 9.7°° | 69.0 ± 5.0°° | 89.1 ± 2.5°° |
| Control | 1.4 ± 2.3 | 5.4 ± 6.8 | 13.5 ± 9.8 | 41.3 ± 11.7 |
| BPS 0.03 mg/kg | 8.9 ± 1.6 | 18.7 ± 5.1 | 40.9 ± 5.5° | 66.9 ± 3.9° |
| BPS 0.1 mg/kg | 8.7 ± 1.5 | 14.5 ± 3.1 | 35.7 ± 4.5 | 60.4 ± 5.1 |
| Each value is average ± standard error. Statistical significant difference: °;p < 0.05, °°p < 0.01 vs. control. | | | | |

### Industrial Applicability

The diabetic angiopathy improving agent of the present invention has a stable chemical structure and has no disadvantage in formulation, and can be applied to oral formulations and a variety of formulations such as various injections, suppositories, and the like.

## Claims

1. A diabetic angiopathy improving agent comprising a prostaglandin I derivative as an active component.

2. The diabetic angiopathy improving agent according to Claim 1, wherein the prostaglandin I derivative is a prostaglandin I₂ derivative.

3. The diabetic angiopathy improving agent according to Claim 1, wherein the prostaglandin I derivative is a 4,8-inter-m-phenylene prostaglandin I derivative represented by the following formula (I) or a pharmacologically acceptable salt thereof. 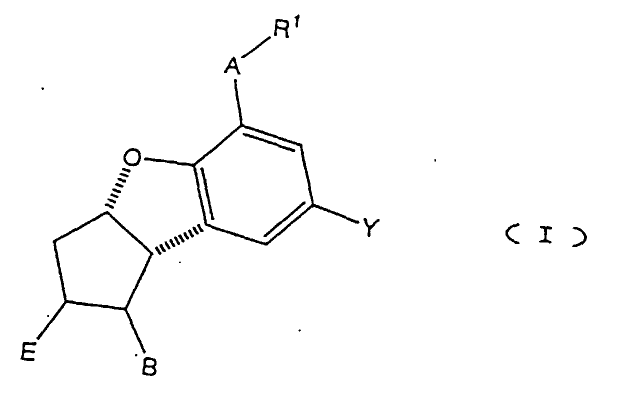 wherein R¹ represents the following:
(A) COOR² wherein R² is:
1) hydrogen or a pharmacologically acceptable cation;
2) straight chain alkyl having 1 to 12 carbon atoms, or branched alkyl having 3 to 14 carbon atoms;
3) -Z-R³
wherein Z is a valence bond or straight chain or branched alkylene represented by CₜH₂ₜ wherein t represents an integer of 1 to 6, and R³ represents cycloalkyl having 3 to 12 carbon atoms or substituted cycloalkyl having 3 to 12 carbon atoms and 1 to 3 substituents R⁴ which is hydrogen or alkyl having 1 to 5 carbon atoms;
4) -(CH₂CH₂O)ₙCH₃
wherein n is an integer of 1 to 5;
5) -Z-Ar¹
wherein Z is defined as the same as the above, and Ar¹ is phenyl, α-naphthyl, β-naphthyl, 2-pyridyl, 3-pyridyl, 4-pyridyl, α-furyl, β-furyl, α-thienyl, β-thienyl or substituted phenyl (wherein a substituent is at least one of chlorine, bromine, fluorine, iodine, trifluoromethyl, alkyl having 1 to 4 carbon atoms, nitro, cyano, methoxy, phenyl, phenoxy, p-acetamidobenzamide, -CH=N-NH-C(=O)-NH₂, -NH-C(=O)-Ph, -NH-C(=O)-CH₃ and -NH-C(=O)-NH₂);
6) -CₜH₂ₜCOOR⁴
wherein CₜH₂ₜ and R⁴ are defined as the same as the above;
7) -CₜH₂ₜN(R⁴)₂
wherein CₜH₂ₜ and R⁴ are defined as the same as the above;
8) -CH(R⁵)-C(=O)-R⁶
wherein R⁵ is hydrogen or benzoyl, and R⁶ is phenyl, p-bromophenyl, p-chlorophenyl, p-biphenyl, p-nitrophenyl, p-benzamidophenyl, or 2-naphthyl;
9 -CₚH₂ₚ-W-R⁷
wherein W is -CH=CH-, -CH=CR⁷ or -C≡C-, and R⁷ is hydrogen or straight chain or branched alkyl or aralkyl having 1 to 30 carbon atoms, and p is an integer of 1 to 5; or
10) -CH(CH₂OR⁸)₂
wherein R⁸ is alkyl or acyl having 1 to 30 carbon atoms;
(B) -CH₂OH;
(C) -C(=O)N(R⁹)₂
wherein R⁹ is hydrogen, straight chain alkyl having 1 to 12 carbon atoms, branched alkyl having 3 to 12 carbon atoms, cycloalkyl having 3 to 12 carbon atoms, cycloalkylalkylene having 4 to 13 carbon atoms, phenyl, substituted phenyl (wherein the substituent is defined as the same as in (A) 5)), aralkyl having 7 to 12 carbon atoms, or -SO₂R¹⁰ wherein R¹⁰ is alkyl having 1 to 10 carbon atoms, cycloalkyl having 3 to 12 carbon atoms, phenyl, substituted phenyl (the substituent is defined as the same as in (A) 5)), or aralkyl having 7 to 12 carbon atoms, two R⁹ groups may be the same or different, and when one of the R⁹ groups is -SO₂R¹⁰, the other R⁹ is not -SO₂R¹⁰; or
(D) -CH₂OTHP (THP is a tetrahydropyranyl group);
A is the following:
1) -(CH₂)ₘ-;
2) -CH=CH-CH₂-;
3) -CH₂-CH=CH-;
4) -CH₂-O-CH₂-;
5) -CH=CH-;
6) -O-CH₂-; or
7) -C≡C-;
wherein m represents an integer of 1 to 3;
Y is hydrogen, alkyl having 1 to 4 carbon atoms, chlorine, bromine, fluorine, formyl, methoxy or nitro;
B is -X-C(R¹¹)(R¹²)OR¹³
wherein R¹¹ is hydrogen, alkyl having 1 to 4 carbon atoms; R¹³ is hydrogen, acyl having 1 to 14 carbon atoms, aroyl having 6 to 15 carbon atoms, tetrahydropyranyl, tetrahydrofuranyl, 1-ethoxyethyl, or t-butyl; X is the following:
1) -CH₂-CH₂-;
2) -CH=CH-; or
3) -C≡C-; and
R¹² is the following:
1) straight chain alkyl having 1 to 12 carbon atoms, or branched alkyl having 3 to 14 carbon atoms; 2) -Z-Ar²
wherein Z is the defined as the same as the above, and Ar² represents phenyl, α-naphthyl, β-naphthyl, or phenyl substituted by at least one chlorine, bromine, fluorine, iodine, trifluoromethyl, alkyl having 1 to 4 carbon atoms, nitro, cyano, methoxy, phenyl or phenoxy;
3) -CₜH₂ₜOR¹⁴
wherein CₜH₂ₜ is defined as the same as the above, and R¹⁴ represents straight chain alkyl having 1 to 6 carbon atoms, branched alkyl having 3 to 6 carbon atoms, phenyl, phenyl substituted by at last one chlorine, bromine, fluorine, iodine, trifluoromethyl, alkyl having 1 to 4 carbon atoms, nitro, cyano, methoxy, phenyl or phenoxy, cyclopentyl, cyclohexyl, or cyclopentyl or cyclohexyl substituted by 1 to 4 straight chain alkyl groups having 1 to 4 carbon atoms;
4) -Z-R³
wherein Z and R³ are defined as the same as the above;
5) -CₜH₂ₜ-CH=C(R¹⁵)R¹⁶
wherein CₜH₂ₜ is defined as the same as the above, and R¹⁵ and R¹⁶ each represent hydrogen, methyl, ethyl, propyl, or butyl; or
6) -CᵤH₂ᵤ-C≡C-R¹⁷
wherein u is an integer of 1 to 7, CᵤH₂ᵤ represents straight chain or branched alkylene, and R¹⁷ represents straight chain alkyl having 1 to 6 carbon atoms;
E is hydrogen or -OR¹⁸
wherein R¹⁸ represents acyl having 1 to 12 carbon atoms, aroyl having 7 to 15 carbon atoms, or R² (wherein R² is defined as the same as the above); and
the formula represents the d, l or dl form.

4. The diabetic angiopathy improving agent according to Claim 3, wherein the 3,4-inter-m-phenylene prostaglandin I derivative is beraprost or a salt thereof.

5. The diabetic angiopathy improving agent according to Claims 1 to 4, wherein diabetic angiopathy is diabetic macroangiopathy or diabetic microangiopathy.

6. A method of preventing or curing diabetic angiopathy comprising administering a prostaglandin I derivative in an effective dose.

7. The method of preventing or curing diabetic angiopathy according to Claim 6, wherein the prostaglandin I derivative is a prostaglandin I₂ derivative.

8. The method of preventing or curing diabetic angiopathy according to Claim 6, wherein the prostaglandin I derivative is a 4,8-inter-m-phenylene prostaglandin I derivative represented by the following formula (I) or a pharmacologically acceptable salt thereof. 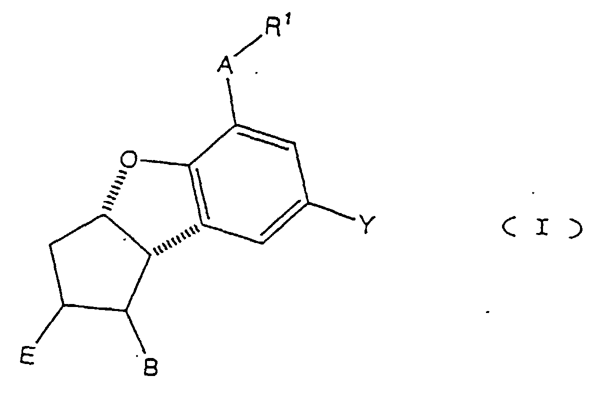 wherein R¹ represents the following:
(A) COOR² wherein R² is:
1) hydrogen or a pharmacologically acceptable cation;
2) straight chain alkyl having 1 to 12 carbon atoms, or branched alkyl having 3 to 14 carbon atoms;
3) -Z-R³
wherein Z is a valence bond or straight chain or branched alkylene represented by CₜH₂ₜ wherein t represents an integer of 1 to 6, and R³ represents cycloalkyl having 3 to 12 carbon atoms or substituted cycloalkyl having 3 to 12 carbon atoms and 1 to 3 substituents R⁴ which is hydrogen or alkyl having 1 to 5 carbon atoms;
4) -(CH₂CH₂O)ₙCH₃
wherein n is an integer of 1 to 5;
5) -Z-Ar¹
wherein Z is defined as the same as the above, and Ar¹ is phenyl, α-naphthyl, β-naphthyl, 2-pyridyl, 3-pyridyl, 4-pyridyl, α-furyl, β-furyl, α-thienyl, β-thienyl or substituted phenyl (wherein a substituent is at least one of chlorine, bromine, fluorine, iodine, trifluoromethyl, alkyl having 1 to 4 carbon atoms, nitro, cyano, methoxy, phenyl, phenoxy, p-acetamidobenzamide, -CH=N-NH-C(=O)-NH₂, -NH-C(=O)-Ph, -NH-C(=O)-CH₃ and -NH-C(=O)-NH₂);
6) -CₜH₂ₜCOOR⁴
wherein CₜH₂ₜ and R⁴ are defined as the same as the above;
7) -CₜH₂ₜN(R⁴)₂
wherein CₜH₂ₜ and R⁴ are defined as the same as the above;
8) -CH(R⁵)-C(=O)-R⁶
wherein R⁵ is hydrogen or benzoyl, and R⁶ is phenyl, p-bromophenyl, p-chlorophenyl, p-biphenyl, p-nitrophenyl, p-benzamidophenyl, or 2-naphthyl;
9) -CₚH₂ₚ-W-R⁷
wherein W is -CH=CH-, -CH=CR⁷ or -C≡C-, and R⁷ is hydrogen or straight chain or branched alkyl or aralkyl having 1 to 30 carbon atoms, and p is an integer of 1 to 5; or
10) -CH(CH₂OR⁸)₂
wherein R⁸ is alkyl or acyl having 1 to 30 carbon atoms;
(B) -CH₂OH;
(C) -C(=O)N(R⁹)₂
wherein R⁹ is hydrogen, straight chain alkyl having 1 to 12 carbon atoms, branched alkyl having 3 to 12 carbon atoms, cycloalkyl having 3 to 12 carbon atoms, cycloalkylalkylene having 4 to 13 carbon atoms, phenyl, substituted phenyl (wherein the substituent is defined as the same as in (A) 5)), aralkyl having 7 to 12 carbon atoms, or -SO₂R¹⁰ wherein R¹⁰ is alkyl having 1 to 10 carbon atoms, cycloalkyl having 3 to 12 carbon atoms, phenyl, substituted phenyl (the substituent is defined as the same as in (A) 5)), or aralkyl having 7 to 12 carbon atoms, two R⁹ groups may be the same or different, and when one of the R⁹ groups is -SO₂R¹⁰, the other R⁹ is not -SO₂R¹⁰; or
(D) -CH₂OTHP (THP is a tetrahydropyranyl group);
A is the following:
1) -(CH₂)ₘ-;
2) -CH=CH-CH₂-;
3) -CH₂-CH=CH-;
4) -CH₂-O-CH₂-;
5) -CH=CH-;
6) -O-CH₂-; or 7) -C=C-;
wherein m represents an integer of 1 to 3;
Y is hydrogen, alkyl having 1 to 4 carbon atoms,
chlorine, bromine, fluorine, formyl, methoxy or nitro;
B is -X-C(R¹¹)(R¹²)OR¹³
wherein R¹¹ is hydrogen, alkyl having 1 to 4 carbon atoms; R¹³ is hydrogen, acyl having 1 to 14 carbon atoms, aroyl having 6 to 15 carbon atoms, tetrahydropyranyl, tetrahydrofuranyl, 1-ethoxyethyl, or t-butyl; X is the following:
1) -CH₂-CH₂-;
2) -CH=CH-; or
3) -C≡C-; and
R¹² is the following:
1) straight chain alkyl having 1 to 12 carbon atoms, or branched alkyl having 3 to 14 carbon atoms;
2) -Z-Ar²
wherein Z is the defined as the same as the above, and Ar² represents phenyl, α-naphthyl, β-naphthyl, or phenyl substituted by at least one chlorine, bromine, fluorine, iodine, trifluoromethyl, alkyl having 1 to 4 carbon atoms, nitro, cyano, methoxy, phenyl or phenoxy;
3) -CₜH₂ₜOR¹⁴
wherein CₜH₂ₜ is defined as the same as the above, and R¹⁴ represents straight chain alkyl having 1 to 6 carbon atoms, branched alkyl having 3 to 6 carbon atoms, phenyl, phenyl substituted by at last one chlorine, bromine, fluorine, iodine, trifluoromethyl, alkyl having 1 to 4 carbon atoms, nitro, cyano, methoxy, phenyl or phenoxy, cyclopentyl, cyclohexyl, or cyclopentyl or cyclohexyl substituted by 1 to 4 straight chain alkyl groups having 1 to 4 carbon atoms;
4) -Z-R³
wherein Z and R³ are defined as the same as the above;
5) -CₜH₂ₜ-CH=C(R¹⁵)R¹⁶
wherein CₜH₂ₜ is defined as the same as the above, and R¹⁵ and R¹⁶ each represent hydrogen, methyl, ethyl, propyl, or butyl; or
6) -CᵤH₂ᵤ-C≡C-R¹⁷
wherein u is an integer of 1 to 7, CᵤH₂ᵤ represents straight chain or branched alkylene, and R¹⁷ represents straight chain alkyl having 1 to 6 carbon atoms;
E is hydrogen or -OR¹⁸
wherein R¹⁸ represents acyl having 1 to 12 carbon atoms, aroyl having 7 to 15 carbon atoms, or R² (wherein R² is defined as the same as the above); and
the formula represents the d, l or dl form.

9. The method of preventing or curing diabetic angiopathy according to Claim 8, wherein the 3,4-inter-m-phenylene prostaglandin I derivative is beraprost or a salt thereof.

10. The method of preventing or curing diabetic angiopathy according to Claims 6 to 9, wherein diabetic angiopathy is diabetic macroangiopathy or diabetic microangiopathy.
